(19) Europäisches Patentamt
European Patent Office
Office européen des brevets

(11) Veröffentlichungsnummer : **0 125 563**
**B1**

(12) # EUROPÄISCHE PATENTSCHRIFT

(45) Veröffentlichungstag der Patentschrift : **27.05.87**

(51) Int. Cl.[4] : **C 07 C 13/28**, C 07 C 43/168, C 07 C 43/21, C 07 C121/46, C 07 C121/00, C 07 C 69/76, C 07 C 69/145, C 07 C 69/24, C 07 C 25/18, C 09 K 19/30, G 02 F 1/13

(21) Anmeldenummer : **84104958.8**

(22) Anmeldetag : **03.05.84**

(54) **Bicyclohexylethane.**

(30) Priorität : **14.05.83 DE 3317597**

(43) Veröffentlichungstag der Anmeldung : **21.11.84 Patentblatt 84/47**

(45) Bekanntmachung des Hinweises auf die Patenterteilung : **27.05.87 Patentblatt 87/22**

(84) Benannte Vertragsstaaten : **CH DE FR GB LI**

(56) Entgegenhaltungen :
**EP-A- 0 056 113**
**EP-A- 0 074 608**
**EP-A- 0 084 194**
**DE-A- 3 225 290**
**Die Akte enthält technische Angaben, die nach dem Eingang der Anmeldung eingereicht wurden und die nicht in dieser Patentschrift enthalten sind.**

(73) Patentinhaber : **Merck Patent Gesellschaft mit beschränkter Haftung**
**Frankfurter Strasse 250**
**D-6100 Darmstadt (DE)**

(72) Erfinder : **Römer, Michael, Dr.**
**Im Grossen Garten 18**
**D-6054 Rodgau 2 (DE)**
Erfinder : **Eidenschink, Rudolf, Dr.**
**Kornblumenstrasse 1**
**D-6115 Münster (DE)**
Erfinder : **Krause, Joachim, Dr.**
**Samuel Morse Strasse 14**
**D-6110 Dieburg (DE)**
Erfinder : **Scheuble, Bernhard, Dr.**
**Am Grenzweg 18**
**D-6146 Alsbach-Hähnlein (DE)**
Erfinder : **Weber, Georg**
**Wilhelm-Leuschner-strasse 38**
**D-6106 Erzhausen (DE)**

**EP 0 125 563 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Bicyclohexylethane der Formel I

$$R^1—Cy—Cy—CH_2CH_2—A—(Cy)_n—R^2 \quad (I)$$

worin

$R^1$ Alkyl,

$R^2$ Alkyl, worin eine oder zwei $CH_2$-Gruppen durch O-Atome ersetzt sein können, F, Cl, Br, CN, —COOAlkyl oder —O—CO-Alkyl,

der eine der Reste $R^1$ bzw. $R^2$ auch H,

A Cy oder unsubstituiertes oder durch ein oder zwei F- und/oder Cl-Atome und/oder $CH_3$-Gruppen oder durch eine CN-Gruppe substituiertes 1,4-Phenylen,

Cy 1,4-Cyclohexylen und

n 0 oder 1

bedeuten, wobei die Alkylgruppen jeweils 1-10 C-Atome enthalten, mit den Maßgaben, daß

a) n 1 bedeutet, wenn A Cy und $R^2$ CN ist und

b) die Verbindungen

1-(4'-trans-Pentyl-trans-bicyclohex-4-yl)-2-(2-methyl-4-pentylphenyl)-ethan

1-(4'-trans-Pentyl-trans-bicyclohex-4-yl)-2-(3-methyl-4-pentylphenyl)-ethan

1-(4'-trans-Pentyl-trans-bicyclohex-4-yl)-2-(4-butoxy-2-methylphenyl)-ethan

1-(4'-trans-Pentyl-trans-bicyclohex-4-yl)-2-(4-butoxy-3-methylphenyl)-ethan

ausgeschlossen sind.

Der Einfachheit halber bedeutet im folgenden « Phe » einen 1,4-Phenylenrest, wobei dieser Rest unsubstituiert oder durch ein oder zwei Fluor- oder Chloratome und/oder $CH_3$-Gruppen oder durch eine CN-Gruppe substituiert sein kann.

In der EP-A-0 084 194 sind zwar bereits neben einer Vielzahl lateral substituierter Flüssigkristallverbindungen auch einige lateral methylierte Biclohexylethane angegeben, die vorteilhaften Eigenschaften der erfindungsgemäßen Stoffklasse der Bicyclohexylethane ist jedoch diesem Dokument nicht zu entnehmen. Gegenüber den aus EP-A-0 056 113 bekannten, strukturell verwandten Cyclohexylcarbonitrilen weisen die Bicyclohexylethane der Formel I breitere flüssigkristalline Bereiche und niedrigere Viskositäten auf.

In der DE-OS-3 148 148 sowie in der EP-OS 0 056 501 sind allgemeine Formeln (1)

$$R^1—A^1—Z^1—(A^2—Z^2)_m—A^3—R^2 \quad (1)$$

angegeben, worin $R^1$ auch $CH_3(CH_2)_n$—, $A^1$ und $A^2$ jeweils auch Cy, $Z^1$ auch eine Valenz, $Z^2$ auch —$CH_2CH_2$—, m auch 1, $A^3$ auch Cy oder 1,4-Phenylen, $R^2$ auch $CH_3(CH_2)_n$, $CH_3(CH_2)_{n-2}COO$—, $CH_3(CH_2)_{n-2}O$—CO— oder $CH_3(CH_2)_{n-1}O$— und n eine Zahl von Null bis 11 sein kann. Ebenda sind weitere allgemeine Formeln (2)

$$R^3—A^4—Z^3(A^5—Z^4)_p—A^6—CN \quad (2)$$

angegeben, worin $R^3$ auch $CH_3(CH_2)_n$—, $A^4$ und $A^5$ jeweils auch Cy, $Z^3$ auch eine Valenz, $Z^4$ auch —$CH_2CH_2$—, p auch 1, $A^6$ auch Cy oder 1,4-Phenylen und n eine Zahl von Null bis 11 sein kann. Es sind dort jedoch weder Teilformeln noch Einzelverbindungen angegeben, die die Gruppe —$CH_2CH_2$— enthalten, wie die Bicyclohexylethane der vorliegenden Formel I. Diese sind gegenüber den beiden genannten Druckschriften daher noch neu, und sie ergeben sich nicht in naheliegender Weise aus dem Stand der Technik, wie er durch die beiden genannten Druckschriften gegeben ist.

Die Verbindungen der Formel I können wie ähnliche, z. B. wie die aus der DE-OS-32 25 290 bekannten Verbindungen, als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindungen der Formel I gelöst.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit breitem flüssigkristallinem Bereich und niedriger Viskosität herstellbar, insbesondere Phasen, die auch bei tiefen Temperaturen flüssigkristallin sind und eine niedrige Viskosität besitzen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen

flüssigkristalline Dielektrika zum überwiegenden Teil zusammengesetzt sind ; es können aber auch Verbindungen der Formel I flüssigkristallinen Basismaterialien aus anderen Verbindungsklassen zugesetzt werden, um beispielsweise die Viskosität oder die Doppelbrechung eines solchen Dielektrikums zu senken und/oder seinen Klärpunkt zu erhöhen. Die Verbindungen der Formel I, insbesondere diejenigen, die Cyclohexanringe mit cis-ständigen Substituenten enthalten, sowie diejenigen, worin $R^2$ H, Cl oder Br bedeutet, eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden niedrigviskose flüssigkristalline Mesophasen in einem für die elektrooptische Verwendung günstig gelegenen Temperaturbereich. Chemisch sind sie sehr stabil.

Gegenstand der Erfindung sind somit die Verbindungen der Formel I sowie ein Verfahren zu ihrer Herstellung, dadurch gekennzeichnet, daß man eine Verbindung, die sonst der Formel I entspricht, aber an Stelle von H-Atomen eine oder mehrere reduzierbare Gruppe(n) und/oder C-C-Bindung(en) enthält, mit einem reduzierenden Mittel behandelt

oder daß man zur Herstellung von Estern der Formel I (worin $R^2$ —COOAlkyl oder —O—COAlkyl bedeutet) eine entsprechende Carbonsäure oder eines ihrer reaktionsfähigen Derivate mit einem entsprechenden Alkohol oder Phenol oder einem seiner reaktionsfähigen Derivate umsetzt,

oder daß man zur Herstellung von Halogenderivaten der Formel I (worin $R^2$ F, Cl oder Br und oder A durch ein oder zwei F- und/oder Cl-Atome substituiertes 1,4-Phenylen bedeutet) mit aromatisch gebundenem Halogen eine entsprechende Aminoverbindung diazotiert und anschließend die Diazoniumgruppe nach an sich bekannten Methoden durch ein Fluor-, Chlor- oder Bromatom ersetzt,

oder daß man zur Herstellung von Nitrilen der Formel I ($R^2$=CN) ein entsprechendes Carbonsäureamid dehydratisiert oder ein entsprechendes Carbonsäurehalogenid mit Sulfamid umsetzt,

oder daß man zur Herstellung von Ethern der Formel I (worin $R^2$ eine Alkylgruppe bedeutet, worin eine oder zwei $CH_2$-Gruppen durch O-Atome ersetzt sind) eine entsprechende Hydroxyverbindung verethert,

und/oder daß man gegebenenfalls eine Chlor- oder Bromverbindung der Formel I (worin $R^2$ Cl oder Br bedeutet und/oder worin A einen durch ein oder zwei Cl-Atome substituierten 1,4-Phenylenrest bedeutet) mit einem Cyanid umsetzt.

Weiterhin ist Gegenstand der Erfindung die Verwendung der Verbindungen der Formel I als Komponenten flüssigkristalliner Dielektrika. Gegenstand der Erfindung sind weiterhin flüssigkristalline Dielektrika mit einem Gehalt an mindestens einer Verbindung der Formel I sowie elektrooptische Anzeigeelemente, die derartige Dielektrika enthalten.

Vor- und nachstehend haben $R^1$, $R^2$, A, Cy, Phe und n die angegebene Bedeutung, sofern nicht ausdrücklich etwas anderes vermerkt ist.

Die Verbindungen der Formel I umfassen dementsprechend Verbindungen der Teilformeln Ia bis Id :

$$R^1\text{—Cy—Cy—}CH_2CH_2\text{—Cy—}R^2 \qquad (Ia)$$

$$R^1\text{—Cy—Cy—}CH_2CH_2\text{—Phe—}R^2 \qquad (Ib)$$

$$R^1\text{—Cy—Cy—}CH_2CH_2\text{—Cy—Cy—}R^2 \qquad (Ic)$$

$$R^1\text{—Cy—Cy—}CH_2CH_2\text{—Phe—Cy—}R^2 \qquad (Id)$$

Von diesen sind diejenigen der Formeln Ia und Ib bevorzugt.

In den Verbindungen der Formel I sowie Ia bis Id können die Alkylreste, worin im Fall $R^2$ auch eine oder zwei $CH_2$-Gruppen durch O-Atome ersetzt sein können, geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, im Fall $R^2$ auch Ethoxy, Propoxy, Butoxy, Pentoxy, Hexoxy, Heptoxy, Methoxymethyl, Ethoxymethyl, Propoxymethyl, Methoxymethoxy, Ethoxymethoxy, ferner Methyl, Octyl, Nonyl, Decyl, Methoxy, Octoxy, Nonoxy oder Decoxy.

Verbindungen der Formel I sowie Ia bis Id mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Heptylpentyl, $R^2$ auch Isopropoxy, 2-Methylpentoxy, 3-Methylpentoxy, 2-Ethylhexoxy, 1-Methylhexoxy, 1-Methylheptoxy.

Im einzelnen bedeutet $R^2$ bevorzugt Alkyl, Alkoxy oder CN, A bevorzugt Cy oder unsubstituiertes 1,4-Phenylen, n bevorzugt 0. Unter den Verbindungen der Formeln I sowie Ia bis Id sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln Ie bis Ih :

$R^1$—Cy—Cy—$CH_2CH_2$—Cy-Alkyl (Ie)

$R^1$—Cy—Cy—$CH_2CH_2$—Phe-Alkyl (If)

$R^1$—Cy—Cy—$CH_2CH_2$—Phe-Alkoxy (Ig)

$R^1$—Cy—Cy—$CH_2CH_2$—Phe—CN (Ih)

In den Verbindungen der Formeln I sowie Ia bis Ih sind diejenigen Stereoisomeren bevorzugt, worin die Substituenten an den Cyclohexylenresten jeweils in trans-Stellung zueinander stehen.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I werden bevorzugt hergestellt, indem man eine Verbindung, die sonst der Formel I entspricht, aber an Stelle von H-Atomen eine oder mehrere reduzierbare Gruppen und/oder C—C-Bindungen enthält, reduziert.

Als reduzierbare Gruppen kommen vorzugsweise Carbonylgruppen in Betracht, insbesondere Ketogruppen, ferner z. B. freie oder veresterte Hydroxygruppen oder aromatisch gebundene Halogenatome. Bevorzugte Ausgangsstoffe für die Reduktion entsprechen der Formel I, enthalten aber an Stelle der Gruppe —$CH_2CH_2$— eine der Gruppen —CO—$CH_2$—, —$CH_2$—CO— oder —CH=CH— und/oder an Stelle eines Cyclohexanrings einen Cyclohexenring.

Bevorzugte Ausgangsstoffe für die Reduktion sind z. B. Ketone der Formeln $R^1$—Cy—Cy—$CH_2$—CO—A—$(Cy)_n$—$R^2$ und $R^1$—Cy—Cy—CO—$CH_2$—A—$(Cy)_n$—$R^2$, insbesondere aber $R^1$—Cy—Cy—$CH_2$—CO—Phe—$(Cy)_n$—$R^2$.

Die zuletzt genannten Ketone sind z. B. erhältlich durch Friedel-Crafts-Reaktion aus den entsprechenden Aromaten der Formel H—Phe—$(Cy)_n$—$R^2$ mit den entsprechenden Säurechloriden der Formel $R^1$—Cy—Cy—$CH_2$—CO—Cl.

Ausgangsstoffe mit Cyclohexenringen sind z. B. solche der Formel

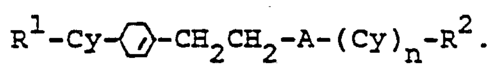

Diese sind beispielsweise erhältlich durch Umsetzung von 4—$R^1$—Cy-cyclohexanonen mit Organometallverbindungen der Formel $LiCH_2CH_2$—A—$(Cy)_n$—$R^2$ oder entsprechenden Grignardverbindungen und anschließende Dehydratisierung der erhaltenen Carbinole.

Weitere bevorzugte Ausgangsstoffe entsprechen der Formel

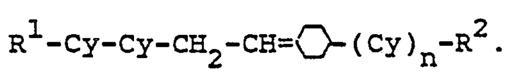

Diese sind z. B. durch Wittig-Reaktion aus Bromiden der Formel $R^1$—Cy—Cy—$CH_2$—$CH_2$—Br mit Triphenylphosphin, dann mit 4—$R^2$—$(Cy)_n$-cyclohexanonen erhältlich.

Die Reduktion erfolgt zweckmäßig durch katalytische Hydrierung bei Temperaturen zwischen etwa 0° und etwa 200° sowie Drucken zwischen etwa 1 und etwa 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan.

Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können. Die genannten Ketone können auch nach den Methoden von Clemmensen (mit Zink, amalgamiertem Zink oder Zinn und Salzsäure, zweckmäßig in wässerig-alkoholischer Lösung oder in heterogener Phase mit Wasser/Benzol oder Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedendem Lösungsmittel wie Diethylenglykol oder Triethylenglykol bei Temperaturen zwischen etwa 100 und 200°) reduziert werden.

Ausgangsstoffe der Formel $R^1$—Cy—Cy—CH=CH—Phe—$(Cy)_n$—$R^2$, die z. B. durch Wittig-Reaktion aus Bromiden der Formel $R^1$—Cy—Cy—$CH_2$Br und Aldehyden der Formel O=CH—Phe—$(Cy)_n$—$R^2$ erhältlich sind, lassen sich auch mit $NaBH_4$ reduzieren, zweckmäßig in einem inerten Lösungsmittel wie Methanol, Ethanol oder Diglyme bei Temperaturen zwischen etwa 0 und 120°.

Ester der Formel I (worin $R^2$—COOAlkyl oder —O—CO-Alkyl bedeutet) können auch durch Veresterung entsprechender Carbonsäuren der Formel $R^3$—COOH [worin $R^3$ (a)

$R^1$—Cy—Cy—$CH_2CH_2$—A—$(Cy)_n$— oder (b) Alkyl bedeutet] (oder ihrer reaktionsfähigen Derivate) mit Alkoholen bzw. Phenolen der Formel $R^4$—OH [worin $R^4$ im Fall (a) Alkyl, im Fall (b) $R^1$—Cy—Cy—$CH_2CH_2$—A—$(Cy)_n$— bedeutet] (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride der Formel $R^3$—CO—O—CO—$CH_3$, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. phenolate der Formel $R^4$—OM in Betracht, in der M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie Dimethylformamid (DMF) oder Phosphorsäure-hexamethyltriamid, Kohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, zum Beispiel Pyridin, Chinolin oder Triethylamin als Lösungsmittel für die Veresterung angewendet werden.

Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen — 50° und + 250°, vorzugsweise zwischen — 20° und + 80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat, Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat under Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa — 25 und + 20°.

Halogenderivate der Formel I (worin $R^2$ F, Cl oder Br und/oder A durch ein oder zwei F- und/oder Cl-Atome substituiertes 1,4-Phenylen bedeutet) mit aromatisch gebundenem Halogen können aus entsprechenden Aminen nach den Methoden der Schiemann-Reaktion (vgl. z. B.).

The Merck Index, 9th Edition, Merck & Co., Inc., Rahway, N.J., USA, 1976, Seite ONR-80) oder der Sandmeyer-Reaktion (vgl. z. B. The Merck Index, 1.c., Seite ONR-79) hergestellt werden.

Zweckmäßig diazotiert man zunächst mit einem Salz oder einem Ester der salpetrigen Säure (wie $NaNO_2$ oder Butylnitrit) in wässerig-saurer Phase, wobei als Säure z. B. HF, HCl, HBr, $H_2SO_4$ oder $HBF_4$ verwendet werden kann, bei Temperaturen zwischen etwa — 20° und + 10°. Dabei kann ein zusätzliches inertes Lösungsmittel zugegen sein, z. B. ein Ether wie THF oder Dioxan oder ein Kohlenwasserstoff wie Toluol oder Xylol.

Zur Herstellung der Fluorverbindungen der Formel I (worin $R^2$ F und/oder A durch ein oder zwei F-Atome substituiertes 1,4-Phenylen bedeutet) diazotiert man zweckmäßig in $HBF_4$. Dabei bilden sich die Diazonium-tetrafluorborate, die bereits bei Temperaturen zwischen etwa 10 und 100° zersetzt werden können. Diazotiert man mit $NaNO_2$ in wasserfreiem HF, so erhält man durch anschließendes Erwärmen direkt die gewünschte Fluorverbindung.

Ein Austausch der Diazoniumgruppe gegen Cl oder Br gelingt bevorzugt in wässeriger Lösung in Gegenwart von $Cu_2Cl_2$ oder $Cu_2Br_2$ bei Temperaturen zwischen 30 und 100°.

Zur Herstellung von Nitrilen der Formel I ($R^2$=CN und/oder A = durch eine CN-Gruppe substituiertes 1,4-Phenylen), insbesondere solchen der Formel Ih, können entsprechende Säureamide, z. B. solche der Formel $R^1$—Cy—Cy—$CH_2CH_2$—A—$(Cy)_n$—$CONH_2$ dehydratisiert werden. Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z. B. als Doppelverbindung mit NaCl), aromatische Sulfonsäuren und Sulfonsäurehalogenide. Man kann dabei in Gegenwart oder Abwesenheit einses inerten Lösungsmittels bei Temperaturen zwischen etwa 0 und 150° arbeiten ; als Lösungsmittel kommen z. B. Basen wie Pyridin oder Triethylamin, aromatische Kohlenwasserstoffe wie Benzol, Toluol oder Xylol oder Amide wie DMF in Betracht.

Alkoxyverbindungen der Formel I ($R^2$ = Alkoxy) sind durch Alkylierung entsprechender Hydroxyverbindungen, vorzugsweise entsprechender Phenole, erhältlich, wobei die Hydroxyverbindung zweckmäßig zunächst in ein entsprechendes Metallderivat, z. B. durch Behandeln mit NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, DMF oder Dimethylsulfoxid oder auch einem Überschuß an wässeriger oder wässerig-alkoholischer NaOH oder KOH bei Temperaturen zwischen etwa 20 und 100°.

Zur Herstellung der Nitrile können auch entsprechende Chlor- oder Bromverbindungen mit einem Cyanid umgesetzt werden, zweckmäßig mit einem Metallcyanid wie NaCN, KCN oder $Cu_2(CN)_2$, z. B. in Gegenwart von Pyridin in einem inerten Lösungsmittel wie DMF oder N-Methylpyrrolidon bei Temperaturen zwischen 20 und 200°.

Zur Herstellung der vorstehend genannten Nitrile kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80 und 150°, vorzugsweise bei etwa 120°. Nach üblicher Aufarbeitung kann man direkt die Nitrile isolieren.

Die erfindungsgemäße Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Biscyclohexylbiphenyle, Phenyl- oder Cyclohexylpyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel III charakterisieren,

$$R^5—L—G—E—R^6 \qquad (III)$$

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | |
|---|---|
| —CH=CH— | —N(O)=N— |
| —CH=CY— | —CH=N(O)— |
| —C≡C— | $—CH_2—CH_2—$ |
| —CO—O— | $—CH_2—O—$ |
| —CO—S— | $—CH_2—S—$ |
| —CH=N— | —COO—Phe—COO— |

oder eine C—C-Einfachbildung, Y Halogen, vorzugsweise Chlor, oder —CN, und $R^5$ und $R^6$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind $R^5$ und $R^6$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrika enthalten etwa 1 bis 60, vorzugsweise 5 bis 40 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z. B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind zum Beispiel in den DE-OS-22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent ; alle Temperaturen sind in Grad Celsius angegeben. « Übliche Aufarbeitung » bedeutet : man gibt Wasser hinzu, extrahiert mit Methylenchlorid, trennt ab, trocknet die organische Phase, dampft ein und reinigt das erhaltene Produkt,

falls erforderlich, durch Chromatographie an Kieselgel.

Beispiel 1

Ein Gemisch aus 36,8 g trans,trans-4-p-Propylbenzoyl-methyl-4'-propyl-bicyclohexyl (erhältlich durch $LiAlH_4$— Reduktion von trans,trans-4'-Propyl-bicyclohexyl-4-carbonsäure zu trans,trans-4-Hydroxymethyl-4'-propyl-bicyclohexyl, Überführung in das Tosylat, Reaktion mit KCN zu trans,trans-4-Cyanmethyl-4'-propyl-bicyclohexyl, Hydrolyse zur Carbonsäure, Reaktion mit $SOCl_2$ zum Säurechlorid und Umsetzung mit Propylbenzol in Gegenwart von $AlCl_3$), 30 g KOH, 50 ml 85 %igem Hydrazin und 500 ml Triethylenglykol wird 1 Stunde auf 120° erwärmt. Man steigert die Temperatur langsam bis zur Zersetzung des gebildeten Hydrazons, kocht noch 4 Stunden, kühlt ab, arbeitet wie üblich auf und erhält trans,trans-4-(2-p-Propylphenyl-ethyl)-4'-propyl-bicyclohexyl, F. 34°, K. 158°.

Analog erhält man durch Reduktion entsprechende Ketone :

trans,trans-4-(2-p-Phenyl-ethyl)-4'-propyl-bicyclohexyl
trans,trans-4-(2-p-Tolyl-ethyl)-4'-propyl-bicyclohexyl
trans,trans-4-(2-p-Ethylphenyl-ethyl)-4'-ethyl-bicyclohexyl
trans,trans-4-(2-p-Ethylphenyl-ethyl)-4'-propyl-bicyclohexyl
trans,trans-4-(2-p-Ethylphenyl-ethyl)-4'-butyl-bicyclohexyl
trans,trans-4-(2-p-Ethylphenyl-ethyl)-4'-pentyl-bicyclohexyl
trans,trans-4-(2-p-Ethylphenyl-ethyl)-4'-hexyl-bicyclohexyl
trans,trans-4-(2-p-Ethylphenyl-ethyl)-4'-heptyl-bicyclohexyl
trans,trans-4-(2-p-Propylphenyl-ethyl)-4'-ethyl-bicyclohexyl
trans,trans-4-(2-p-Propylphenyl-ethyl)-4'-butyl-bicyclohexyl
trans,trans-4-(2-p-Propylphenyl-ethyl)-4'-pentyl-bicyclohexyl
trans,trans-4-(2-p-Propylphenyl-ethyl)-4'-hexyl-bicyclohexyl
trans,trans-4-(2-p-Propylphenyl-ethyl)-4'-heptyl-bicyclohexyl
trans,trans-4-(2-p-Butylphenyl-ethyl)-4'-ethyl-bicyclohexyl
trans,trans-4-(2-p-Butylphenyl-ethyl)-4'-propyl-bicyclohexyl
trans,trans-4-(2-p-Butylphenyl-ethyl)-4'-butyl-bicyclohexyl
trans,trans-4-(2-p-Butylphenyl-ethyl)-4'-pentyl-bicyclohexyl
trans,trans-4-(2-p-Butylphenyl-ethyl)-4'-hexyl-bicyclohexyl
trans,trans-4-(2-p-Butylphenyl-ethyl)-4'-heptyl-bicyclohexyl
trans,trans-4-(2-p-Pentylphenyl-ethyl)-4'-ethyl-bicyclohexyl
trans,trans-4-(2-p-Pentylphenyl-ethyl)-4'-propyl-bicyclohexyl
trans,trans-4-(2-p-Pentylphenyl-ethyl)-4'-butyl-bicyclohexyl
trans,trans-4-(2-p-Pentylphenyl-ethyl)-4'-pentyl-bicyclohexyl
trans,trans-4-(2-p-Pentylphenyl-ethyl)-4'-hexyl-bicyclohexyl
trans,trans-4-(2-p-Pentylphenyl-ethyl)-4'-heptyl-bicyclohexyl
trans,trans-4-(2-p-Hexylphenyl-ethyl)-4'-ethyl-bicyclohexyl
trans,trans-4-(2-p-Hexylphenyl-ethyl)-4'-propyl-bicyclohexyl
trans,trans-4-(2-p-Hexylphenyl-ethyl)-4'-butyl-bicyclohexyl
trans,trans-4-(2-p-Hexylphenyl-ethyl)-4'-pentyl-bicyclohexyl
trans,trans-4-(2-p-Hexylphenyl-ethyl)-4'-hexyl-bicyclohexyl
trans,trans-4-(2-p-Hexylphenyl-ethyl)-4'-heptyl-bicyclohexyl
trans,trans-4-(2-p-Heptylphenyl-ethyl)-4'-ethyl-bicyclohexyl
trans,trans-4-(2-p-Heptylphenyl-ethyl)-4'-propyl-bicyclohexyl
trans,trans-4-(2-p-Heptylphenyl-ethyl)-4'-butyl-bicyclohexyl
trans,trans-4-(2-p-Heptylphenyl-ethyl)-4'-pentyl-bicyclohexyl
trans,trans-4-(2-p-Heptylphenyl-ethyl)-4'-hexyl-bicyclohexyl
trans,trans-4-(2-p-Heptylphenyl-ethyl)-4'-heptyl-bicyclohexyl
trans,trans-4-(2-p-Methoxymethylphenyl-ethyl)-4'-ethyl-bicyclohexyl
trans,trans-4-(2-p-Methoxymethylphenyl-ethyl)-4'-propyl-bicyclohexyl
trans,trans-4-(2-p-Methoxymethylphenyl-ethyl)-4'-butyl-bicyclohexyl
trans,trans-4-(2-p-Methoxymethylphenyl-ethyl)-4'-pentyl-bicyclohexyl
trans,trans-4-(2-p-Methoxymethylphenyl-ethyl)-4'-hexyl-bicyclohexyl
trans,trans-4-(2-p-Methoxymethylphenyl-ethyl)-4'-heptyl-bicyclohexyl
trans,trans-4-(2-p-Methoxyphenyl-ethyl)-4'-ethyl-bicyclohexyl
trans,trans-4-(2-p-Methoxyphenyl-ethyl)-4'-propyl-bicyclohexyl
trans,trans-4-(2-p-Methoxyphenyl-ethyl)-4'-butyl-bicyclohexyl
trans,trans-4-(2-p-Methoxyphenyl-ethyl)-4'-pentyl-bicyclohexyl
trans,trans-4-(2-p-Methoxyphenyl-ethyl)-4'-hexyl-bicyclohexyl
trans,trans-4-(2-p-Methoxyphenyl-ethyl)-4'-heptyl-bicyclohexyl
trans,trans-4-(2-p-Ethoxyphenyl-ethyl)-4'-ethyl-bicyclohexyl
trans,trans-4-(2-p-Ethoxyphenyl-ethyl)-4'-propyl-bicyclohexyl

trans,trans-4-(2-p-Ethoxyphenyl-ethyl)-4'-butyl-bicyclohexyl
trans,trans-4-(2-p-Ethoxyphenyl-ethyl)-4'-pentyl-bicyclohexyl
trans,trans-4-(2-p-Ethoxyphenyl-ethyl)-4'-hexyl-bicyclohexyl
trans,trans-4-(2-p-Ethoxyphenyl-ethyl)-4'-heptyl-bicyclohexyl
trans,trans-4-(2-p-Propoxyphenyl-ethyl)-4'-ethyl-bicyclohexyl
trans,trans-4-(2-p-Propoxyphenyl-ethyl)-4'-propyl-bicyclohexyl
trans,trans-4-(2-p-Propoxyphenyl-ethyl)-4'-butyl-bicyclohexyl
trans,trans-4-(2-p-Propoxyphenyl-ethyl)-4'-pentyl-bicyclohexyl
trans,trans-4-(2-p-Propoxyphenyl-ethyl)-4'-hexyl-bicyclohexyl
trans,trans-4-(2-p-Propoxyphenyl-ethyl)-4'-heptyl-bicyclohexyl
trans,trans-4-(2-p-Butoxyphenyl-ethyl)-4'-ethyl-bicyclohexyl
trans,trans-4-(2-p-Butoxyphenyl-ethyl)-4'-propyl-bicyclohexyl
trans,trans-4-(2-p-Butoxyphenyl-ethyl)-4'-butyl-bicyclohexyl
trans,trans-4-(2-p-Butoxyphenyl-ethyl)-4'-pentyl-bicyclohexyl
trans,trans-4-(2-p-Butoxyphenyl-ethyl)-4'-hexyl-bicyclohexyl
trans,trans-4-(2-p-Butoxyphenyl-ethyl)-4'-heptyl-bicyclohexyl
trans,trans-4-(2-p-Methoxymethoxyphenyl-ethyl)-4'-ethyl-bicyclohexyl
trans,trans-4-(2-p-Methoxymethoxyphenyl-ethyl)-4'-propyl-bicyclohexyl
trans,trans-4-(2-p-Methoxymethoxyphenyl-ethyl)-4'-butyl-bicyclohexyl
trans,trans-4-(2-p-Methoxymethoxyphenyl-ethyl)-4'-pentyl-bicyclohexyl
trans,trans-4-(2-p-Methoxymethoxyphenyl-ethyl)-4'-hexyl-bicyclohexyl
trans,trans-4-(2-p-Methoxymethoxyphenyl-ethyl)-4'-heptyl-bicyclohexyl
trans,trans-4-[2-p-(trans-4-Ethylcyclohexyl)-phenyl-ethyl]-4'-ethyl-bicyclohexyl
trans,trans-4-[2-p-(trans-4-Ethylcyclohexyl)-phenyl-ethyl]-4'-propyl-bicyclohexyl
trans,trans-4-[2-p-(trans-4-Ethylcyclohexyl)-phenyl-ethyl]-4'-butyl-bicyclohexyl
trans,trans-4-[2-p-(trans-4-Ethylcyclohexyl)-phenyl-ethyl]-4'-pentyl-bicyclohexyl
trans,trans-4-[2-p-(trans-4-Ethylcyclohexyl)-phenyl-ethyl]-4'-hexyl-bicyclohexyl
trans,trans-4-[2-p-(trans-4-Ethylcyclohexyl)-phenyl-ethyl]-4'-heptyl-bicyclohexyl
trans,trans-4-[2-p-(trans-4-Propylcyclohexyl)-phenyl-ethyl]-4'-ethyl-bicyclohexyl
trans,trans-4-[2-p-(trans-4-Propylcyclohexyl)-phenyl-ethyl]-4'-propyl-bicyclohexyl
trans,trans-4-[2-p-(trans-4-Propylcyclohexyl)-phenyl-ethyl]-4'-butyl-bicyclohexyl
trans,trans-4-[2-p-(trans-4-Propylcyclohexyl)-phenyl-ethyl]-4'-pentyl-bicyclohexyl
trans,trans-4-[2-p-(trans-4-Propylcyclohexyl)-phenyl-ethyl]-4'-hexyl-bicyclohexyl
trans,trans-4-[2-p-(trans-4-Propylcyclohexyl)-phenyl-ethyl]-4'-heptyl-bicyclohexyl
trans,trans-4-[2-p-(trans-4-Butylcyclohexyl)-phenyl-ethyl]-4'-ethyl-bicyclohexyl
trans,trans-4-[2-p-(trans-4-Butylcyclohexyl)-phenyl-ethyl]-4'-propyl-bicyclohexyl
trans,trans-4-[2-p-(trans-4-Butylcyclohexyl)-phenyl-ethyl]-4'-butyl-bicyclohexyl
trans,trans-4-[2-p-(trans-4-Butylcyclohexyl)-phenyl-ethyl]-4'-pentyl-bicyclohexyl
trans,trans-4-[2-p-(trans-4-Butylcyclohexyl)-phenyl-ethyl]-4'-hexyl-bicyclohexyl
trans,trans-4-[2-p-(trans-4-Butylcyclohexyl)-phenyl-ethyl]-4'-heptyl-bicyclohexyl
trans,trans-4-[2-p-(trans-4-Pentylcyclohexyl)-phenyl-ethyl]-4'-ethyl-bicyclohexyl
trans,trans-4-[2-p-(trans-4-Pentylcyclohexyl)-phenyl-ethyl]-4'-propyl-bicyclohexyl
trans,trans-4-[2-p-(trans-4-Pentylcyclohexyl)-phenyl-ethyl]-4'-butyl-bicyclohexyl
trans,trans-4-[2-p-(trans-4-Pentylcyclohexyl)-phenyl-ethyl]-4'-pentyl-bicyclohexyl
trans,trans-4-[2-p-(trans-4-Pentylcyclohexyl)-phenyl-ethyl]-4'-hexyl-bicyclohexyl
trans,trans-4-[2-p-(trans-4-Pentylcyclohexyl)-phenyl-ethyl]-4'-heptyl-bicyclohexyl
trans,trans-4-[2-p-(trans-4-Hexylcyclohexyl)-phenyl-ethyl]-4'-ethyl-bicyclohexyl
trans,trans-4-[2-p-(trans-4-Hexylcyclohexyl)-phenyl-ethyl]-4'-propyl-bicyclohexyl
trans,trans-4-[2-p-(trans-4-Hexylcyclohexyl)-phenyl-ethyl]-4'-butyl-bicyclohexyl
trans,trans-4-[2-p-(trans-4-Hexylcyclohexyl)-phenyl-ethyl]-4'-pentyl-bicyclohexyl
trans,trans-4-[2-p-(trans-4-Hexylcyclohexyl)-phenyl-ethyl]-4'-hexyl-bicyclohexyl
trans,trans-4-[2-p-(trans-4-Hexylcyclohexyl)-phenyl-ethyl]-4'-heptyl-bicyclohexyl
trans,trans-4-[2-p-(trans-4-Heptylcyclohexyl)-phenyl-ethyl]-4'-ethyl-bicyclohexyl
trans,trans-4-[2-p-(trans-4-Heptylcyclohexyl)-phenyl-ethyl]-4'-propyl-bicyclohexyl
trans,trans-4-[2-p-(trans-4-Heptylcyclohexyl)-phenyl-ethyl]-4'-butyl-bicyclohexyl
trans,trans-4-[2-p-(trans-4-Heptylcyclohexyl)-phenyl-ethyl]-4'-pentyl-bicyclohexyl
trans,trans-4-[2-p-(trans-4-Heptylcyclohexyl)-phenyl-ethyl]-4'-hexyl-bicyclohexyl
trans,trans-4-[2-p-(trans-4-Heptylcyclohexyl)-phenyl-ethyl]-4'-heptyl-bicyclohexyl.

## Beispiel 2

Eine Lösung von 39,6 g trans,trans-4-p-Propylbenzoyl-methyl-4'-pentyl-bicyclohexyl (erhältlich aus trans,trans-4'-Pentyl-bicyclohexyl-4-acetylchlorid und Propylbenzol/$AlCl_3$) in 500 ml Ethylacetat wird an 5 g 10 %iger Pd-Kohle bei 20° und 5 bar hydriert. Man filtriert, dampft ein und erhält trans,trans-4-(2-p-Propylphenyl-ethyl)-4'-pentyl-bicyclohexyl.

Analog sind auch die übrigen in Beispiel 1 genannten Verbindungen erhältlich.

Beispiel 3

Eine Lösung von 34 g 1-(2-p-Methoxyphenyl-ethyl)-4-(trans-4-propyl-cyclohexyl)-cyclohexen [erhältlich durch Reaktion von 2-p-Methoxyphenyl-ethylmagnesium-bromid mit 4-(trans-4-Propyl-cyclohexyl)-cylohexanon und nachfolgende Hydrolyse zu 1-(2-p-Methoxyphenyl-ethyl)-4-(trans-4-propylcyclohexyl)-cyclohexanol und Dehydratisierung mit p-Toluolsulfonsäure in siedendem Toluol] in 600 ml THF wird an 5 g PdO bei 40° und 1 bar bis zum Stillstand hydriert. Man filtriert, dampft ein, nimmt in 200 ml siedendem Methanol auf und gießt die erhaltene heiße Lösung in eine siedende Lösung von 70 g Thioharnstoff in 300 ml Methanol. Das Gemisch wird auf 0° abgekühlt, die ausgefallene Thioharnstoff-Einschlußverbindung abfiltriert und mit 350 ml Petrolether (Kp. 40-60°) ausgekocht. Der ungelöste Rückstand wird mit 350 ml 2 N wässeriger Kalilauge 30 Min. auf 50° erwärmt. Man säuert die erhaltene Lösung mit 10 %iger $H_2SO_4$ an und filtriert das ausgefallene trans,trans-4-(2-p-Methoxy-phenylethyl)-4'-propyl-bicyclohexyl ab.

Analog sind auch die übrigen in Beispiel 1 genannten Verbindungen erhältlich.

Beispiel 4

Ein Gemisch von 36,3 g trans,trans-4-(2-p-Cyanphenyl-vinyl)-4'-pentyl-bicyclohexyl (erhältlich durch Wittig-Reaktion aus 4-Brommethyl-4'-pentyl-bicyclohexyl und p-Cyanbenzaldehyd), 3,7 g $NaBH_4$ und 350 ml Methanol wird 4 Stunden gekocht. Nach üblicher Aufarbeitung erhält man trans,trans-4-(2-p-Cyanphenyl-ethyl)-4'-pentyl-bicyclohexyl.

Analog erhält man durch Reduktion der entsprechenden Vinylverbindungen :

trans,trans-4-(2-p-Cyanphenyl-ethyl)-4'-ethyl-bicyclohexyl
trans,trans-4-(2-p-Cyanphenyl-ethyl)-4'-propyl-bicyclohexyl
trans,trans-4-(2-p-Cyanphenyl-ethyl)-4'-butyl-bicyclohexyl
trans,trans-4-(2-p-Cyanphenyl-ethyl)-4'-hexyl-bicyclohexyl
trans,trans-4-(2-p-Cyanphenyl-ethyl)-4'-heptyl-bicyclohexyl.

Beispiel 5

Man kocht 35,6 g p-[2-(trans,trans-4'-Propyl-bicyclohexyl-4-yl)-ethyl]-benzoesäure (erhältlich durch Wittig-Reaktion aus trans,trans-4-Brommethyl-4'-propyl-bicyclohexyl und Terephthalaldehydsäure zu p-[2-(trans,trans-4'-Propyl-bicyclohexyl-4-yl)-vinyl]-benzoesäure und Hydrierung) 1 Stunde mit 24 g $SOCl_2$, dampft ein, löst das erhaltene rohe Säurechlorid in 300 ml Toluol, versetzt mit 8 ml Pyridin und 13 g (—)-2-Octanol und kocht 2 Stunden. Nach Abkühlen und üblicher Aufarbeitung erhält man p-[2-(trans,trans-4'-Propyl-bicyclohexyl-4-yl)-ethyl]-benzoesäure-((—)-2-octylester).

Analog sind durch Veresterung erhältlich :

p-[2-(trans,trans-4'-Ethyl-bicyclohexyl-4-yl)-ethyl]-benzoesäure-ethylester
p-[2-(trans,trans-4'-Ethyl-bicyclohexyl-4-yl)-ethyl]-benzoesäure-propylester
p-[2-(trans,trans-4'-Ethyl-bicyclohexyl-4-yl)-ethyl]-benzoesäure-butylester
p-[2-(trans,trans-4'-Propyl-bicyclohexyl-4-yl)-ethyl]-benzoesäure-ethylester
p-[2-(trans,trans-4'-Propyl-bicyclohexyl-4-yl)-ethyl]-benzoesäure-propylester
p-[2-(trans,trans-4'-Propyl-bicyclohexyl-4-yl)-ethyl]-benzoesäure-butylester
p-[2-(trans,trans-4'-Butyl-bicyclohexyl-4-yl)-ethyl]-benzoesäure-ethylester
p-[2-(trans,trans-4'-Butyl-bicyclohexyl-4-yl)-ethyl]-benzoesäure-propylester
p-[2-(trans,trans-4'-Butyl-bicyclohexyl-4-yl)-ethyl]-benzoesäure-butylester
p-[2-(trans,trans-4'-Pentyl-bicyclohexyl-4-yl)-ethyl]-benzoesäure-ethylester
p-[2-(trans,trans-4'-Pentyl-bicyclohexyl-4-yl)-ethyl]-benzoesäure-propylester
p-[2-(trans,trans-4'-Pentyl-bicyclohexyl-4-yl)-ethyl]-benzoesäure-butylester
p-[2-(trans,trans-4'-Hexyl-bicyclohexyl-4-yl)-ethyl]-benzoesäure-ethylester
p-[2-(trans,trans-4'-Hexyl-bicyclohexyl-4-yl)-ethyl]-benzoesäure-propylester
p-[2-(trans,trans-4'-Hexyl-bicyclohexyl-4-yl)-ethyl]-benzoesäure-butylester
p-[2-(trans,trans-4'-Heptyl-bicyclohexyl-4-yl)-ethyl]-benzoesäure-ethylester
p-[2-(trans,trans-4'-Heptyl-bicyclohexyl-4-yl)-ethyl]-benzoesäure-propylester
p-[2-(trans,trans-4'-Heptyl-bicyclohexyl-4-yl)-ethyl]-benzoesäure-butylester

Beispiel 6

Man erhitzt 32,8 g trans,trans-4-(2-p-Hydroxyphenylethyl)-4'-propyl-bicyclohexyl und 30 g $CH_3COO$-Na in 100 ml Acetanhydrid 1 Stunde auf 80°, kühlt ab, arbeitet wie üblich auf und erhält trans,trans-4-(2-p-Acetoxyphenylethyl)-4'-propyl-bicyclohexyl.

Analog erhält man durch Veresterung

trans,trans-4-(2-p-Acetoxyphenylethyl)-4'-ethyl-bicyclohexyl
trans,trans-4-(2-p-Acetoxyphenylethyl)-4'-butyl-bicyclohexyl
trans,trans-4-(2-p-Acetoxyphenylethyl)-4'-pentyl-bicyclohexyl
trans,trans-4-(2-p-Acetoxyphenylethyl)-4'-hexyl-bicyclohexyl
trans,trans-4-(2-p-Acetoxyphenylethyl)-4'-heptyl-bicyclohexyl
trans,trans-4-(2-p-Propionyloxyphenylethyl)-4'-ethyl-bicyclohexyl
trans,trans-4-(2-p-Propionyloxyphenylethyl)-4'-propyl-bicyclohexyl
trans,trans-4-(2-p-Propionyloxyphenylethyl)-4'-butyl-bicyclohexyl
trans,trans-4-(2-p-Propionyloxyphenylethyl)-4'-pentyl-bicyclohexyl
trans,trans-4-(2-p-Propionyloxyphenylethyl)-4'-hexyl-bicyclohexyl
trans,trans-4-(2-p-Propionyloxyphenylethyl)-4'-heptyl-bicyclohexyl
trans,trans-4-(2-p-Butyryloxyphenylethyl)-4'-ethyl-bicyclohexyl
trans,trans-4-(2-p-Butyryloxyphenylethyl)-4'-propyl-bicyclohexyl
trans,trans-4-(2-p-Butyryloxyphenylethyl)-4'-butyl-bicyclohexyl
trans,trans-4-(2-p-Butyryloxyphenylethyl)-4'-pentyl-bicyclohexyl
trans,trans-4-(2-p-Butyryloxyphenylethyl)-4'-hexyl-bicyclohexyl
trans,trans-4-(2-p-Butyryloxyphenylethyl)-4'-heptyl-bicyclohexyl.

## Beispiel 7

Zu einer Suspension von 39,7 g trans,trans-4-[2-(3-Amino-4-propylphenyl)-ethyl]-4'-pentyl-bicyclohexyl (erhältlich durch Nitrieren von trans,trans-4-p-Propyl-benzoylmethyl-4'-pentyl-bicyclohexyl und anschließende Hydrierung der erhaltenen rohen 3-Nitroverbindung an Pd in THF) in 180 ml Dioxan tropft man bei 15-20° unter Rühren 180 ml 35 %ige wässerige Tetrafluorborsäurelösung und dann bei 0° eine Lösung von 7,2 g $NaNO_2$ in 25 ml Wasser. Man rührt 6 Stunden nach, wobei man die Temperatur auf 15° steigen läßt. Nach üblicher Aufarbeitung (Extraktion mit Toluol, Filtration über Kieselgel) erhält man trans,trans-4-[2-(3-Fluor-4-propylphenyl)-ethyl]-4'-pentyl-bicyclohexyl.

Analog sind aus den entsprechenden Aminoverbindungen erhältlich :

trans,trans-4-(2-o-Fluorphenyl-ethyl)-4'-propyl-bicyclohexyl
trans,trans-4-(2-p-Fluorphenyl-ethyl)-4'-ethyl-bicyclohexyl
trans,trans-4-(2-p-Fluorphenyl-ethyl)-4'-propyl-bicyclohexyl
trans,trans-4-(2-p-Fluorphenyl-ethyl)-4'-butyl-bicyclohexyl
trans,trans-4-(2-p-Fluorphenyl-ethyl)-4'-pentyl-bicyclohexyl
trans,trans-4-(2-p-Fluorphenyl-ethyl)-4'-hexyl-bicyclohexyl
trans,trans-4-(2-p-Fluorphenyl-ethyl)-4'-heptyl-bicyclohexyl

## Beispiel 8

Zu einer Suspension von 35,5 g trans,trans-4-(2-p-Aminophenyl-ethyl)-4'-pentyl-bicyclohexyl [erhältlich durch Nitrierung von trans,trans-4-(2-Phenylethyl)-4'-pentyl-bicyclohexyl zu 4-(2-p-Nitrophenyl-ethyl)-4'-pentyl-bicyclohexyl und Hydrierung an Pd] in 150 ml 15 %iger wässeriger Bromwasserstoffsäure gibt man bei — 5° innerhalb 0,5 Stunden eine Lösung von 7,2 g $NaNO_2$ in 25 ml Wasser. Die so erhaltene Lösung wird bei 0-5° innerhalb 1 Stunde zu einer Cu(I)-salzlösung hinzugetropft, die aus 25 g Kupfersulfat, 15,4 g NaBr, 6,3 g $Na_2SO_3$, 100 ml Wasser und 40 ml 32 %iger Bromwasserstoffsäure hergestellt worden ist. Nach halbstündigem Erhitzen auf 40° wird abgekühlt, mit $CH_2Cl_2$ extrahiert und wie üblich aufgearbeitet. Man erhält trans,trans-4-(2-p-Bromphenyl-ethyl)-4'-pentyl-bicyclohexyl.

Analog sind durch Sandmeyer-Reaktion aus den entsprechenden Aminoverbindungen erhältlich :

trans,trans-4-(2-p-Chlorphenyl-ethyl)-4'-ethyl-bicyclohexyl
trans,trans-4-(2-p-Chlorphenyl-ethyl)-4'-propyl-bicyclohexyl
trans,trans-4-(2-p-Chlorphenyl-ethyl)-4'-butyl-bicyclohexyl
trans,trans-4-(2-p-Chlorphenyl-ethyl)-4'-pentyl-bicyclohexyl
trans,trans-4-(2-p-Chlorphenyl-ethyl)-4'-hexyl-bicyclohexyl
trans,trans-4-(2-p-Chlorphenyl-ethyl)-4'-heptyl-bicyclohexyl
trans,trans-4-(2-p-Bromphenyl-ethyl)-4'-ethyl-bicyclohexyl
trans,trans-4-(2-p-Bromphenyl-ethyl)-4'-propyl-bicyclohexyl
trans,trans-4-(2-p-Bromphenyl-ethyl)-4'-butyl-bicyclohexyl
trans,trans-4-(2-p-Bromphenyl-ethyl)-4'-hexyl-bicyclohexyl
trans,trans-4-(2-p-Bromphenyl-ethyl)-4'-heptyl-bicyclohexyl.

## Beispiel 9

Eine Lösung von 45,7 g trans,trans-4-[2-p-(trans-4-Chlorcarbonylcyclohexyl)-phenyl-ethyl]-4'-propyl-bicyclohexyl und 8 g Sulfamid in 500 ml Tetramethylensulfon wird 4 Stunden auf 120° erhitzt, eingedampft und wie üblich aufgearbeitet. Man erhält trans,trans-4-[2-p-(trans-4-Cyan-cyclohexyl)-phenyl-ethyl]-4'-propyl-bicyclohexyl.

Analog erhält man aus den entsprechenden Säurechloriden :

trans,trans-4-[2-p-(trans-4-cyancyclohexyl)-phenyl-ethyl]-4'-ethyl-bicyclohexyl
trans,trans-4-[2-p-(trans-4-cyancyclohexyl)-phenyl-ethyl]-4'-butyl-bicyclohexyl
trans,trans-4-[2-p-(trans-4-cyancyclohexyl)-phenyl-ethyl]-4'-pentyl-bicyclohexyl
trans,trans-4-[2-p-(trans-4-cyancyclohexyl)-phenyl-ethyl]-4'-hexyl-bicyclohexyl
trans,trans-4-[2-p-(trans-4-cyancyclohexyl)-phenyl-ethyl]-4'-heptyl-bicyclohexyl
trans,trans-4-[2-(trans, trans-4'-cyan-bicyclohexyl-4-yl)-ethyl]-4'-ethyl-bicyclohexyl
trans,trans-4-[2-(trans, trans-4'-cyan-bicyclohexyl-4-yl)-ethyl]-4'-propyl-bicyclohexyl
trans,trans-4-[2-(trans, trans-4'-cyan-bicyclohexyl-4-yl)-ethyl]-4'-butyl-bicyclohexyl
trans,trans-4-[2-(trans, trans-4'-cyan-bicyclohexyl-4-yl)-ethyl]-4'-pentyl-bicyclohexyl
trans,trans-4-[2-(trans, trans-4'-cyan-bicyclohexyl-4-yl)-ethyl]-4'-hexyl-bicyclohexyl
trans,trans-4-[2-(trans, trans-4'-cyan-bicyclohexyl-4-yl)-ethyl]-4'-heptyl-bicyclohexyl.

## Beispiel 10

Ein Gemisch aus 41,9 g trans,trans-4-(2-p-Bromphenyl-ethyl)-4'-pentyl-bicyclohexyl, 10 g $Cu_2(CN)_2$, 120 ml Pyridin und 60 ml N-Methylpyrrolidon wird 2 Stunden auf 150° erhitzt. Man kühlt ab, gibt eine Lösung von 120 g Eisen(III)chlorid-hexahydrat in 600 ml 20 %iger Salzsäure hinzu, erwärmt 1,5 Stunden unter Rühren auf 70°, arbeitet wie üblich auf und erhält trans,trans-4-(2-p-Cyanphenyl-ethyl)-4'-pentyl-bicyclohexyl.

Analog sind die in den Beispielen 4, 9 und 10 angegebenen Nitrile erhältlich, ferner

trans,trans-4-[2-(2-Cyan-4-propylphenyl)-ethyl]-4'-propyl-bicyclohexyl
trans,trans-4-[2-(2-3-Difluor-4-cyanphenyl)-ethyl]-4'-propyl-bicyclohexyl
trans,trans-4-[2-(2-Methyl-4-cyanphenyl)-ethyl]-4'-propyl-bicyclohexyl.

## Beispiel 11

Ein Gemisch von 35,6 g trans,trans-4-(2-p-Hydroxy-phenyl-ethyl)-4'-pentyl-bicyclohexyl (erhältlich durch Wittig-Reaktion aus trans,trans-4-Brommethyl-4'-pentylbicyclohexyl und p-Hydroxybenzaldehyd zu trans,trans-4-(2-p-Hydroxyphenyl-vinyl)-4'-pentyl-bicyclohexyl und Hydrierung), 6,9 g $K_2CO_3$, 25 g Hexyljodid und 250 ml DMF wird unter Rühren 16 Stunden auf 80° erhitzt, dann abgekühlt und wie üblich aufgearbeitet. Man erhält trans,trans-4-(2-p-Hexoxyphenyl-ethyl)-4'-pentyl-bicyclohexyl.

Analog erhält man durch Veretherung die in Beispiel 1 genannten Ether sowie folgende Verbindungen :

trans,trans-4-(2-p-Pentoxyphenyl-ethyl)-4'-propyl-bicyclohexyl
trans,trans-4-(2-p-Pentoxyphenyl-ethyl)-4'-butyl-bicyclohexyl
trans,trans-4-(2-p-Pentoxyphenyl-ethyl)-4'-pentyl-bicyclohexyl
trans,trans-4-(2-p-Hexoxyphenyl-ethyl)-4'-propyl-bicyclohexyl
trans,trans-4-(2-p-Hexoxyphenyl-ethyl)-4'-butyl-bicyclohexyl
trans,trans-4-(2-p-Hexoxyphenyl-ethyl)-4'-pentyl-bicyclohexyl
trans,trans-4-(2-p-Heptoxyphenyl-ethyl)-4'-propyl-bicyclohexyl
trans,trans-4-(2-p-Heptoxyphenyl-ethyl)-4'-butyl-bicyclohexyl
trans,trans-4-(2-p-Heptoxyphenyl-ethyl)-4'-pentyl-bicyclohexyl
trans,trans-4-(2-p-Octoxyphenyl-ethyl)-4'-propyl-bicyclohexyl
trans,trans-4-(2-p-Octoxyphenyl-ethyl)-4'-butyl-bicyclohexyl
trans,trans-4-(2-p-Octoxyphenyl-ethyl)-4'-pentyl-bicyclohexyl
trans,trans-4-(2-p-Nonoxyphenyl-ethyl)-4'-propyl-bicyclohexyl
trans,trans-4-(2-p-Nonoxyphenyl-ethyl)-4'-butyl-bicyclohexyl
trans,trans-4-(2-p-Nonoxyphenyl-ethyl)-4'-pentyl-bicyclohexyl
trans,trans-4-(2-p-Decoxyphenyl-ethyl)-4'-propyl-bicyclohexyl
trans,trans-4-(2-p-Decoxyphenyl-ethyl)-4'-butyl-bicyclohexyl
trans,trans-4-(2-p-Decoxyphenyl-ethyl)-4'-pentyl-bicyclohexyl

Die folgenden Beispiele betreffen erfindungsgemäße Dielektrika mit einem Gehalt an mindestens einer Verbindung der Formel I :

## Beispiel A

Ein Zusatz von trans,trans-4-(2-p-Propylphenyl-ethyl)-4'-propyl-bicyclohexyl (« A ») erhöht den Klärpunkt eines Gemisches (« M ») von 29 % p-trans-4-Propylcyclohexyl-benzonitril, 41 % p-trans-4-Pentylcyclohexyl-benzonitril und 30 % p-trans-4-Heptylcyclohexyl-benzonitril wie folgt :

100 % "M" K. 52°

90 % "M" + 10 % "A" K. 61°

80 % "M" + 20 % "A" K. 70°

70 % "M" + 30 % "A" K. 79°.

Beispiel B

Ein Zusatz von « A » erhöht den Klärpunkt eines Gemischs (« N ») von 24 % p-trans-4-Propylcyclohexyl-benzonitril, 36 % p-trans-4-Pentylcyclohexyl-benzonitril, 25 % p-trans-4-Heptylcyclohexyl-benzonitril und 15 % 4-Cyan-4'-(trans-4-pentylcyclohexyl)-biphenyl wie folgt :

100 % "N" K. 71°

90 % "N" + 10 % "A" K. 78°

80 % "N" + 20 % "A" K. 85°

70 % "N" + 30 % "A" K. 93°.

Beispiel.C

Ein Gemisch aus

17 % « A »
15 % p-trans-4-Propylcyclohexyl-benzonitril
27 % trans-1-p-Ethylphenyl-4-propylcyclohexan
10 % trans-2-p-Ethoxyphenyl-4-propylcyclohexan
7 % 4-Cyan-4'-(trans-4-pentylcyclohexyl)-biphenyl
10 % 4-(trans-4-Pentylcyclohexyl)-4'-(trans-4-propylcyclohexyl)-biphenyl
8 % p-trans-4-Propylcyclohexyl-benzoesäure-(p-propylphenylester)
6 % p-trans-4-Pentylcyclohexyl-benzoesäure-(p-propylphenylester)

zeigt K. 85°.

Beispiel D

Ein Gemisch aus

19 % « A »
15 % p-trans-4-Propylcyclohexyl-benzonitril
12 % p-trans-4-Butylcyclohexyl-benzonitril
14 % trans-1-p-Ethylphenyl-4-propylcyclohexan
10 % trans-1-p-Ethoxyphenyl-4-propylcyclohexan
6 % 4-Cyan-4'-(trans-4-pentylcyclohexyl)-biphenyl
4 % 4,4'-Bis-(trans-4-propylcyclohexyl)-biphenyl
6 % 4-(trans-4-Pentylcyclohexyl)-4'-(trans-4-propylcyclohexyl)-biphenyl
8 % p-trans-4-Propylcyclohexyl-benzoesäure-(p-propylphenylester)
6 % p-trans-4-Pentylcyclohexyl-benzoesäure-(p-propylphenylester)

zeigt K. 100°.

**Patentansprüche**

1. Bicyclohexylethane der Formel I

$$R^1—Cy—Cy—CH_2CH_2—A—(Cy)_n—R^2 \quad (I)$$

worin

$R^1$ Alkyl,

$R^2$ Alkyl, worin eine oder zwei $CH_2$-Gruppen durch O-Atome ersetzt sein können, F, Cl, Br, CN, —COOAlkyl oder —O—CO-Alkyl,

der eine der Reste $R^1$ bzw. $R^2$ auch H,

A Cy oder unsubstituiertes oder durch ein oder zwei F- und/oder Cl-Atome und/oder $CH_3$-Gruppen oder durch eine CN-Gruppe substituiertes 1,4-Phenylen,

Cy 1,4-Cyclohexylen und

n 0 oder 1

bedeuten, wobei die Alkylgruppen jeweils 1-10 C-Atome enthalten, mit den Maßgaben, daß

a) n 1 bedeutet, wenn A Cy und $R^2$ CN ist und

b) die Verbindungen

1-(4'-trans-Pentyl-trans-bicyclohex-4-yl)-2-(2-methyl-4-pentylphenyl)-ethan
1-(4'-trans-Pentyl-trans-bicyclohex-4-yl)-2-(3-methyl-4-pentylphenyl)-ethan
1-(4'-trans-Pentyl-trans-bicyclohex-4-yl)-2-(4-butoxy-2-methylphenyl)-ethan
1-(4'-trans-Pentyl-trans-bicyclohex-4-yl)-2-(4-butoxy-3-methylphenyl)-ethan

ausgeschlossen sind.

2. Verfahren zur Herstellung von Bicyclohexylethanen der Formel I nach Anspruch 1, dadurch gekennzeichnet, daß man eine Verbindung, die sonst der Formel I entspricht, aber an Stelle von H-Atomen eine oder mehrere reduzierbare Gruppe(n) und/oder zusätzliche C-C-Bindung(en) enthält, mit einem reduzierenden Mittel behandelt, oder daß man zur Herstellung von Estern der Formel I (worin $R^2$ —COOAlkyl oder —O—COAlkyl bedeutet) eine entsprechende Carbonsäure oder eines ihrer reaktionsfähigen Derivate mit einem entsprechenden Alkohol oder Phenol oder einem seiner reaktionsfähigen Derivate umsetzt, oder daß man zur Herstellung von Halogenderivaten der Formel I (worin $R^2$ F, Cl oder Br und/oder A durch ein oder zwei F- und/oder Cl-Atome substituiertes 1,4-Phenylen bedeutet) mit aromatisch gebundenem Halogen eine entsprechende Aminoverbindung diazotiert und anschließend die Diazoniumgruppe nach an sich bekannten Methoden durch ein Fluor-, Chlor- oder Bromatom ersetzt, oder daß man zur Herstellung von Nitrilen der Formel I ($R^2$=CN) ein entsprechendes Carbonsäureamid dehydratisiert oder ein entsprechendes Carbonsäurehalogenid mit Sulfamid umsetzt, oder daß man zur Herstellung von Ethern der Formel I (worin $R^2$ eine Alkylgruppe bedeutet, worin eine oder zwei $CH_2$-Gruppen durch O-Atome ersetzt sind) eine entsprechende Hydroxyverbindung verethert und/oder daß man gegebenenfalls eine Chlor- oder Bromverbindung der Formel I (worin $R^2$ Cl oder Br bedeutet und/oder worin A einen durch ein oder zwei Cl-Atome substituierten 1,4-Phenylenrest bedeutet) mit einem Cyanid umsetzt.

3. Verwendung der Verbindungen der Formel I nach Anspruch 1 als Komponenten flüssigkristalliner Dielektrika.

4. Flüssigkristallines Dielektrikum mit einem Gehalt an mindestens einer Verbindung der Formel I nach Anspruch 1.

5. Elektrooptisches Anzeigeelement, dadurch gekennzeichnet, daß es ein Dielektrikum nach Anspruch 4 enthält.

## Claims

1. Bicyclohexylethanes of the formula I

$$R^1—Cy—Cy—CH_2CH_2—A—(Cy)_n—R^2 \quad (I)$$

wherein

$R^1$ is alkyl,

$R^2$ is alkyl, in which one or two $CH_2$ groups can be replaced by O atoms, or is F, Cl, Br, CN, —COOalkyl or O—CO-alkyl,

and one of the radicals $R^1$ and $R^2$ can also be H,

A is Cy, or 1,4-phenylene which is unsubstituted or substituted by one or two F and/or Cl atoms and/or $CH_3$ groups or by a CN group,

Cy is 1,4-cyclohexylene and

n is 0 or 1,

the alkyl groups in each case containing 1-10 C atoms, with the proviso that

a) n is 1 if A is Cy and $R^2$ is CN and

b) the compounds

1-(4'-trans-pentyl-trans-bicyclohex-4-yl)-2-(2-methyl-4-pentylphenyl)-ethane
1-(4'-trans-pentyl-trans-bicyclohex-4-yl)-2-(3-methyl-4-pentylphenyl)-ethane
1-(4'-trans-pentyl-trans-bicyclohex-4-yl)-2-(4-butoxy-2-methylphenyl)-ethane

1-(4'-trans-pentyl-trans-bicyclohex-4-yl)-2-(4-butoxy-3-methylphenyl)-ethane are excluded.

2. Process for the preparation of bicyclohexylethanes of the formula I according to Claim 1, characterised in that a compound which otherwise corresponds to the formula I but contains one or more reducible group(s) and/or additional C—C bond(s) instead of H atoms, is treated with a reducing agent, or in that, for the preparation of esters of the formula I (wherein $R^2$ is —COOalkyl or —O—COalkyl), a corresponding carboxylic acid or one of its reactive derivatives is reacted with a corresponding alcohol or phenol or one of its reactive derivatives, or in that, for the preparation of halogen derivatives of the formula I (wherein $R^2$ is F, Cl or Br and/or A is 1,4-phenylene which is substituted by one or two F and/or Cl atoms) with aromatically bonded halogen, a corresponding amino compound is diazotised and the diazonium group is then replaced by a fluorine, chlorine or bromine atom by methods which are known per se, or in that, for the preparation of nitriles of the formula I ($R^2$=CN), a corresponding carboxylic acid amide is dehydrated or a corresponding carboxylic acid halide is reacted with sulfamide, or in that, for the preparation of ethers of the formula I (wherein $R^2$ is an alkyl group in which one or two $CH_2$ groups are replaced by O atoms), a corresponding hydroxy compound is etherified, and/or in that, if appropriate, a chlorine or bromine compound of the formula I (wherein $R^2$ is Cl or Br and/or wherein A denotes a 1,4-phenylene radical which is substituted by one or two Cl atoms) is reacted with a cyanide.

3. Use of the compounds of the formula I according to Claim 1 as components of liquid crystal dielectrics.

4. Liquid crystal dielectric containing at least one compound of the formula I according to Claim 1.

5. Electrooptical display element, characterised in that it contains a dielectric according to Claim 4.

**Revendications**

1. Bicyclohexyléthanes de formule I

$$R^1—Cy—Cy—CH_2CH_2—A—(Cy)_n—R^2 \quad (I)$$

dans laquelle

$R^1$ représente un groupe alkyle,

$R^2$ représente un groupe alkyle dans lequel un ou deux groupes $CH_2$ peuvent être remplacés par des atomes d'oxygène, F, Cl, Br, CN, un groupe —COO-alkyle ou —O—CO-alkyle,

l'un des symboles $R^1$ et $R^2$ pouvant également représenter l'hydrogène,

A représente le groupe Cy ou un groupe 1,4-phénylène non substitué ou substitué par 1 ou 2 atomes de F et/ou Cl et/ou groupes $CH_3$ ou par un groupe CN,

Cy représente le groupe 1,4-cyclohexylène, et

n est égal à 0 ou 1,

les groupes alkyle contenant chacun 1 à 10 atomes de carbone, avec les restrictions suivantes :

a) n est égal à 1 lorsque A représente Cy et $R^2$ représente CN, et

b) les composés :

1-(4'-trans-pentyl-trans-bicyclohexa-4-yl)-2-(2-méthyl-4-pentylphényl)-éthane,
1-(4'-trans-pentyl-trans-bicyclohexa-4-yl)-2-(3-méthyl-4-pentylphényl)-éthane,
1-(4'-trans-pentyl-trans-bicyclohexa-4-yl)-2-(4-butoxy-2-méthylphényl)-éthane,
1-(4'-trans-pentyl-trans-bicyclohexa-4-yl)-2-(4-butoxy-3-méthylphényl)-éthane

sont exclus.

2. Procédé de préparation des bicyclohexyléthanes de formule I selon la revendication 1, caractérisé en ce que l'on traite un composé répondant par ailleurs à la formule I, mais contenant un ou plusieurs groupes réductibles et/ou une ou plusieurs liaisons C—C supplémentaires à la place d'atomes d'hydrogène, par un agent réducteur, ou bien, pour préparer les esters de formule I (dans laquelle $R^2$ représente un groupe —COO-alkyle ou —O—CO-alkyle) on fait réagir un acide carboxylique correspondant ou l'un de ses dérivés réactifs avec un alcool ou phénol correspondant ou l'un de leurs dérivés réactifs, ou bien, pour préparer les dérivés halogénés de formule I (dans laquelle $R^2$ représente F, Cl ou Br et/ou A représente un groupe 1,4-phénylène substitué par 1 ou 2 atomes de F et/ou Cl) portant des halogènes à liaisons aromatiques, on diazote un composé aminé correspondant puis on remplace le groupe diazonium, selon des procédés connus en soi, par un atome de fluor, de chlore ou de brome, ou bien, pour préparer les nitriles de formule I ($R^2$=CN), on déshydrate un carboxamide correspondant ou on fait réagir un halogénure d'acide carboxylique correspondant avec le sulfonamide, ou bien, pour préparer les éthers de formule I (dans laquelle $R^2$ représente un groupe alkyle dans lequel 1 ou 2 groupes $CH_2$ sont remplacés par des atomes d'oxygène), on éthérifie un composé hydroxylé correspondant, et/ou le cas échéant, on fait réagir un composé chloré ou bromé de formule I (dans laquelle $R^2$ représente Cl ou Br et/ou dans laquelle A représente un groupe 1,4-phénylène substitué par 1 ou 2 atomes de chlore) avec un cyanure.

3. Utilisation des composés de formule I selon la revendication 1, en tant que composants de diélectriques à cristaux liquides.

4. Diélectrique à cristaux liquides contenant au moins un composé de formule I selon la revendication 1.

5. Elément d'affichage électro-optique, caractérisé en ce qu'il contient un diélectrique selon la revendication 4.